# EUROPEAN PATENT SPECIFICATION

(11) **EP 2 271 334 B1**
(45) Date of publication and mention of the grant of the patent: **12.08.2015**
(21) Application number: 09724901.5
(22) Date of filing: 25.03.2009
(51) Int. Cl.: A61K 31/352, A61P 11/00, A61P 13/12, A61P 1/16

(54) **METHODS OF TREATING FIBROTIC DISORDERS**
VERFAHREN ZUR BEHANDLUNG VON FIBROTISCHEN ERKRANKUNGEN
PROCÉDÉS DE TRAITEMENT DE TROUBLES FIBROTIQUES

(30) Priority: 25.03.2008 US 39146
(43) Date of publication of application: 12.01.2011
(73) Proprietor: Paloma Pharmaceuticals, Inc., Buffalo Grove, IL 60089 (US)
(72) Inventor: SHERRIS, David, Illinois 60089 (US)
(74) Representative: Duffy, Assumpta Dympna
(86) International application number: PCT/US2009/038285
(87) International publication number: WO 2009/120799

(56) References cited:
- WO-A2-2007/101247
- "Presentations highlight Palomid 529 as a first-class Akt inhibitor showing efficacy in models of macular degeneration, diabetic retinopathy and retinal scarring caused by retinal detachment", , 8 February 2008 (2008-02-08), XP002646838, Jamaica Plain Retrieved from the Internet: URL:http://www.palomapharma.com/dfiles/new s/8%20February%202008%20news%20entry.pdf [retrieved on 2011-07-01]

## Description

### BACKGROUND OF THE INVENTION

Pathological fibrosis is described by an aberrant expansion, firming, and/or scarring of tissue. It is characterized by an excess deposition of extracellular matrix components including collagen. Fibrosis typically results from a state of chronic inflammation in which inflammation, tissue remodeling and repair processes occur simultaneously. Despite having distinct etiological and clinical manifestations, most chronic fibrotic disorders have in common a persistent irritant or stimuli including persistent infections, autoimmune reactions, allergic responses, chemical insults, radiation, and tissue injury. The irritant or stimuli sustains the production of growth factors, proteolytic enzymes, angiogenic factors and fibrogenic cytokines, which stimulate the deposition of connective tissue elements that progressively remodel and destroy normal tissue architecture. In some diseases, such as idiopathic pulmonary fibrosis, liver cirrhosis, cardiovascular fibrosis, systemic sclerosis and nephritis, extensive tissue remodeling and fibrosis can ultimately lead to organ failure and death.

### BRIEF SUMMARY OF THE INVENTION

The present invention relates to compositions and methods for treating fibrotic disorders. The current invention is directed to a benzo[c]chromen-6-one compound, namely SG 00529, and pharmaceutical compositions thereof that demonstrate a therapeutic benefit in diseases involving fibrosis.

The present invention relates to methods of administering a therapeutically effective amount of the composition comprising SG00529 to a subject in need thereof. In one aspect, the targeted subject has been diagnosed with a fibrotic disorder.

Other features and advantages of the invention will be apparent from the following detailed description of embodiments thereof.

### BRIEF DESCRIPTION OF THE DRAWINGS

Figures 1A-1F are photographs of control attached retinas. A and B are light micrographs of H & E stained paraffin sections illustrating the normal retinal morphology in both the vehicle (A) and drug (B) injected normal eyes. C and D are laser scanning confocal images of attached retinal regions from within the eyes of animals with 3 or 7 day detachments and receiving an intraocular injection of either the vehicle (C,D) or drug (E,F);
Figures 2A-2D are laser scanning confocal images of retinal sections of detached retinas labeled with anti-vimentin (Müller cells, green), anti-BrdU (dividing cells, red) and isolectin B4 (microglia and macrophages, blue);
Figure 3 is a bar graph illustrating the number of Müller cells proliferating after retinal detachment;
Figure 4 is a bar graph illustrating the number of subretinal glial scars after retinal detachment;
Figure 5 is a bar graph illustrating the length of subretinal glial scars after retinal detachment;
Figure 6 is a bar graph illustrating the number of immune-related cells after retinal detachment;
Figure 7 is a bar graph illustrating the average thickness of the outer nuclear layer (ONL) after detachment;
Figure 8A is a photograph of a retinal section of a non-detached retina;
Figures 8B and 8C are photographs of a retinal section of a detached retina without treatment with compound;
Figure 8D-8F are photographs of a retinal section of a detached retina treated with compound;

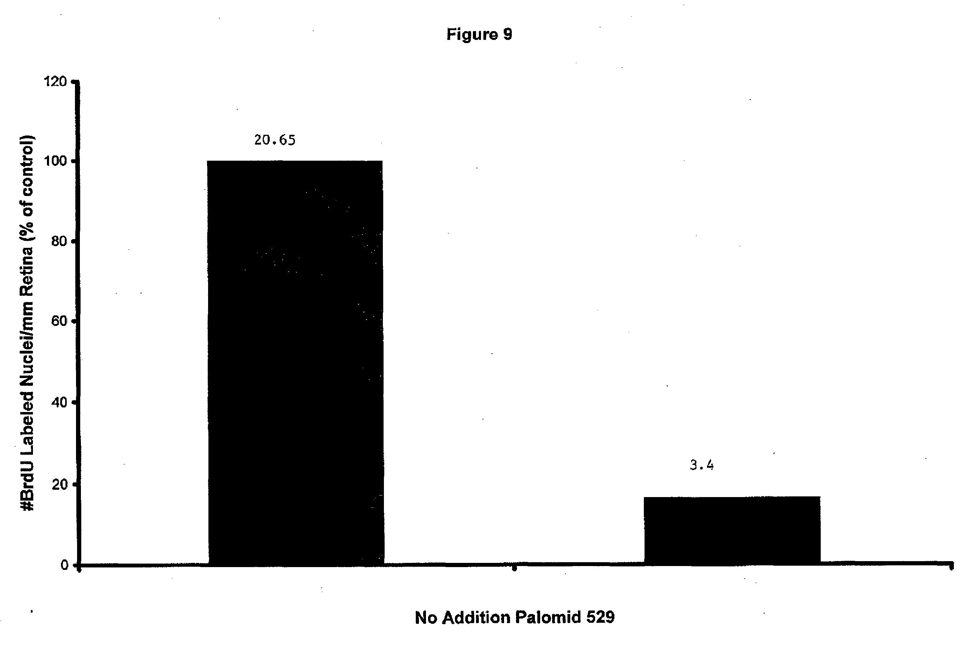

Figure 9 is a bar graph of the number of dividing cells per mm of detached retina;
Figure 10A is a photograph of a retinal section of a non-detached retina;
Figure 10B is a photograph of a retinal section of a detached retina without treatment with compound showing subretinal fibrosis;
Figure 10C is a photograph of a retinal section of a detached retina treated with compound showing no fibrosis;
Figure 11 is a photograph of a Western Blot showing the effect of Palomid 529 on mediators of differentiation of human lung fibroblasts; and
Figure 12 is a photograph of a Western Blot showing the effect of Palomid 529 on signal transduction in human lung fibroblasts.

### DETAILED DESCRIPTION OF THE INVENTION

Most chronic fibrotic disorders have in common a persistent irritant or stimuli including persistent infections, autoimmune reactions, allergic responses, chemical insults, radiation, and tissue injury. The irritant or stimuli sustains the production of growth factors, proteolytic enzymes, angiogenic factors and fibrogenic cytokines, which stimulate the deposition of connective tissue components that replace normal parenchymal tissue. In some diseases, such as idiopathic pulmonary fibrosis, liver cirrhosis, cardiovascular fibrosis, systemic sclerosis and nephritis, extensive tissue remodelling and fibrosis can ultimately lead to organ failure and death (Table 1). Wynn, TA, "Cellular and Molecular Mechanisms of Fibrosis", J Pathol, 214:199-210 (2008).

**Table 1. Major tissues affected by fibrosis and possible contributing factors**

| |
|---|
| ▪ *Liver* - Viral hepatitis, schistosomiasis, and alcoholism are leading causes of cirrhosis worldwide. |
| ▪ *Lung* - The interstitial lung diseases (ILDs) include a diverse set of disorders in which pulmonary inflammation and fibrosis are the final common pathological manifestations. There are more than 150 different causes of ILDs, including sarcoidosis, silicosis, drug reactions and infections, as well as collagen vascular diseases, such as rheumatoid arthritis and systemic sclerosis (scleroderma). Idiopathic pulmonary fibrosis, the most common type of ILD, has no known cause |
| ▪ *Kidney disease* - Diabetes damages and scars the kidneys, which can lead to a progressive loss of function. Untreated hypertension can contribute |
| ▪ *Heart and vascular disease -* Following a heart attack, scar tissue can impair the ability of the heart to pump blood. Hypertension, atherosclerosis and restenosis also contribute |
| ▪ *Eye -* Macular degeneration, retinal and vitreal retinopathy can lead to blindness |
| ▪ *Skin -* Including keloids and hypertrophic scars. Systemic sclerosis and scleroderma, bums and genetic factors may also contribute |
| ▪ *Pancreas -* Poorly understood but possible autoimmune/hereditary causes |
| ▪ *Intestine -* Crohn's disease/inflammatory bowel disease. Pathogenic orgnanisms |
| ▪ *Brain -* Alzheimer's disease, AIDS |
| ▪ *Bone marrow -* Cancer and ageing |
| ▪ *Multi-organ fibrosis* - (a) Due to surgical complications; scar tissue can form between internal organs, causing contracture, pain and, in some cases, infertility; (b) chemotherapeutic drug-induced fibrosis; (c) radiation-induced fibrosis as a result of cancer therapy/accidental exposure; (d) mechanical injuries |

In this invention we describe examples of fibrosis that involve the eye and the lung with the understanding that the induction of fibrosis occurs through a series of events in a similar manner in a variety of tissues. We describe that benzo[c]chromen-6-one compounds and pharmaceutical compositions thereof demonstrate a therapeutic benefit in diseases involving fibrosis. We further describe that inhibition of the Akt/mTOR signal transduction pathway by benzo[c]chromen-6-one compounds and pharmaceutical compositions thereof demonstrate a therapeutic benefit in diseases involving fibrosis.

The present invention relates to compositions and methods for preventing and/or treating diseases associated with fibrosis. The current invention relates to a series of chemical compositions that demonstrate therapeutic benefit in fibrotic diseases. The instant invention is directed to a benzo[c]chromen-6-one derivative, namely SG 00529, that demonstrates its effect on pulmonary fibrotic diseases.

The term "derivative" is understood by those skilled in the art. For example, a derivative can be understood as a chemical compound that is produced from another compound of similar structure in one or more steps, such as illustrated in Table 2 (*infra)* for benzo[c]chromen-6-one.

The present invention relates to a therapeutic formulation comprising one or more compositions useful in the treatment of fibrotic disorders. Fibrotic disorders are due to the abnormal formation of an extracellular matrix. Examples of fibrotic disorders include pulmonary fibrosis, systemic sclerosis, scleroderma, proliferative vitreoretinopathy, hepatic cirrhosis and mesangial proliferative cell disorders. Further examples of fibrotic disorders include cystic fibrosis of the pancreas and lungs, endomyocardial fibrosis, idiopathic pulmonary fibrosis of the lung, mediastinal fibrosis, myleofibrosis, retroperitoneal fibrosis, nephrogenic systemic fibrosis, progressive massive fibrosis (a complication of coal workers' pneumoconiosis) and injection fibrosis (which can occur as a complication of intramuscular injections, especially in children). Further fibrosis-associated conditions include diffuse parenchymal lung disease, post-vasectomy pain syndrome, tuberculosis (which can cause fibrosis of the lungs), sickle-cell anemia (which can cause fibrosis of the spleen) and rheumatoid arthritis.

Hepatic cirrhosis is characterized by the increase in extracellular matrix constituents resulting in the formation of a hepatic scar. Hepatic cirrhosis can cause diseases such as cirrhosis of the liver. An increased extracellular matrix resulting in a hepatic scar can also be caused by viral infection such as hepatitis.

Mesangial disorders are brought about by abnormal proliferation of mesangial cells. Mesangial hyperproliferative cell disorders include various human renal diseases, such as glomerulonephritis, diabetic nephropathy, malignant nephrosclerosis, thrombotic microangiopathy syndromes, transplant rejection, and glomerulopathies.

Described herein are compounds of Formula I: 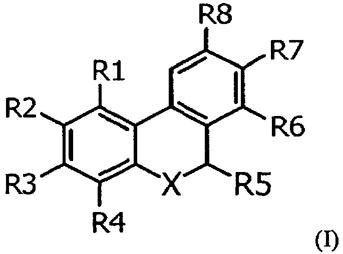 where,
**R1** = H or alkyl;
**R2** = H, OH, O-alkyl, amino, O-heterocyc, O-aryl, O-substituted alkyl, where substitution is e.g. halo, aryl, or heteroaryl, O-Ac, O-PO3, O-SO3, or OSO2NH2; **R3** = H, OH, O-alkyl, O-CH2Aryl, O-CH2heteroaryl, O-alkylaryl, O-acyl, or nitro;
**R4** = H, Alkyl, CH2Aryl, substituted alkyl, OH, O-alkyl, O-aryl, OCH2Aryl, OCH2Heteroaryl, O-Acyl, OPO3, OSO3, or OSO2NH2;
**R5 =** H, Oxo, aryl, hydroxyl, alkyl, or O-alkyl;
**R6** = H;
**R7** = H, Acyl, substituted alkyl, where substitution is e.g. hydroxyl or sulfamoyl, alkyl, O-alkyl, or O-substituted alkyl where substitution is O-PO3 or OSO3; **R8** = H; and
**X** = O, N, or S.

Also described herein are compounds of Formula II: 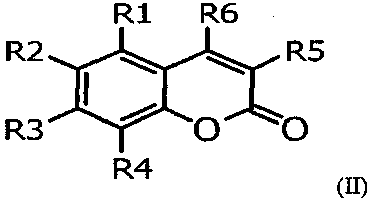 where,
**R1** = H or alkyl;
**R2** = H, O-alkyl, OH, amino, O-heterocyc, O-aryl, O-substituted alkyl where substitution is e.g. halo, aryl, or heteroaryl, O-Ac, O-PO3, O-SO3, or OSO2NH2;
**R3** = H, O-alkyl, O-substituted alkyl where substitution is aryl or heteroaryl, OH, O-acyl, or nitro;
**R4** = H, Alkyl, CH2Aryl, substituted alkyl, O), O-alkyl, O-aryl, OCH2Aryl, OCH2Heteroaryl, O-Acyl, OPO3, OSO3, or OSO2NH2;
**R5** = H, Aryl, heteroaryl or subsitituted alkyl; and
**R6** = H, Alkyl, or Aryl.

Also described herein are compounds of Formula III: 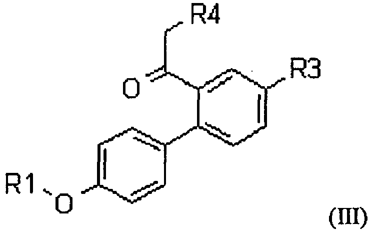 where,
**R1** = alkyl or H;
**R2** = alkyl or H;
**R3** = Acetyl; and
**R4** = H or Alkyl.

Also described herein are compounds of Formula IV: 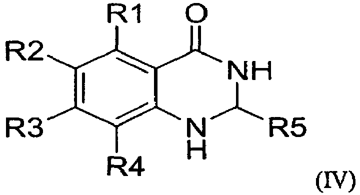 where,
**R1** = H or F;
**R2** = H or nitro;
**R3** = H;
**R4 =** H; and
**R5** = alkyl, substituted alkyl or aryl.

Also described herein are benzo[c]chromen-6-one derivatives having the following structure depicted in Table 2:

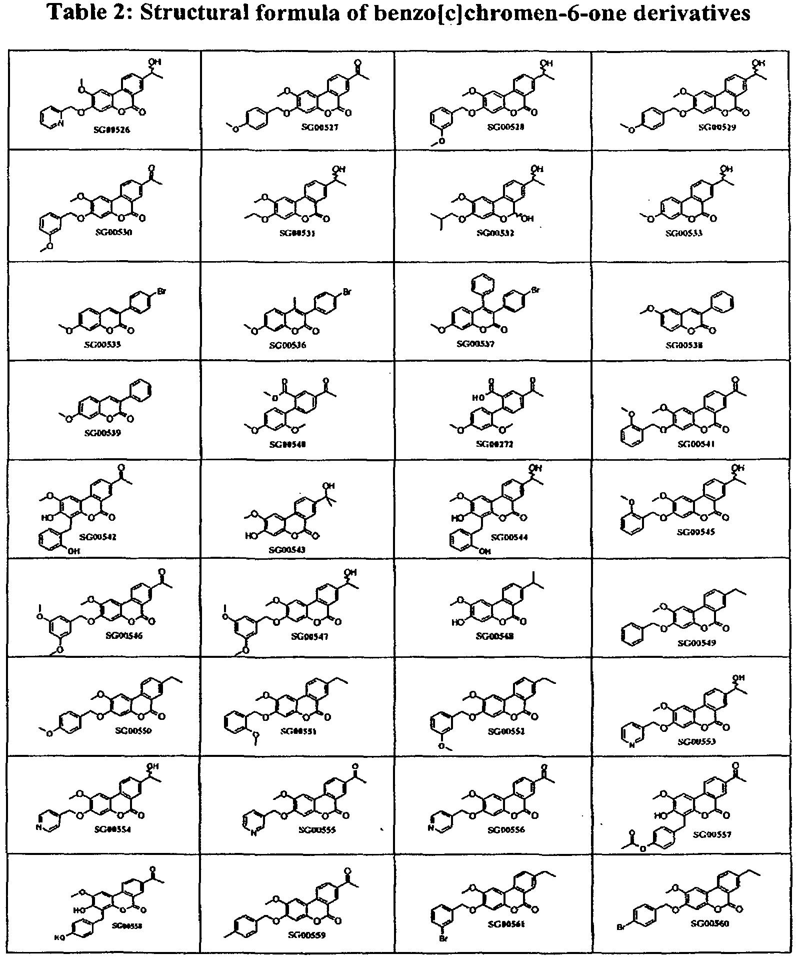

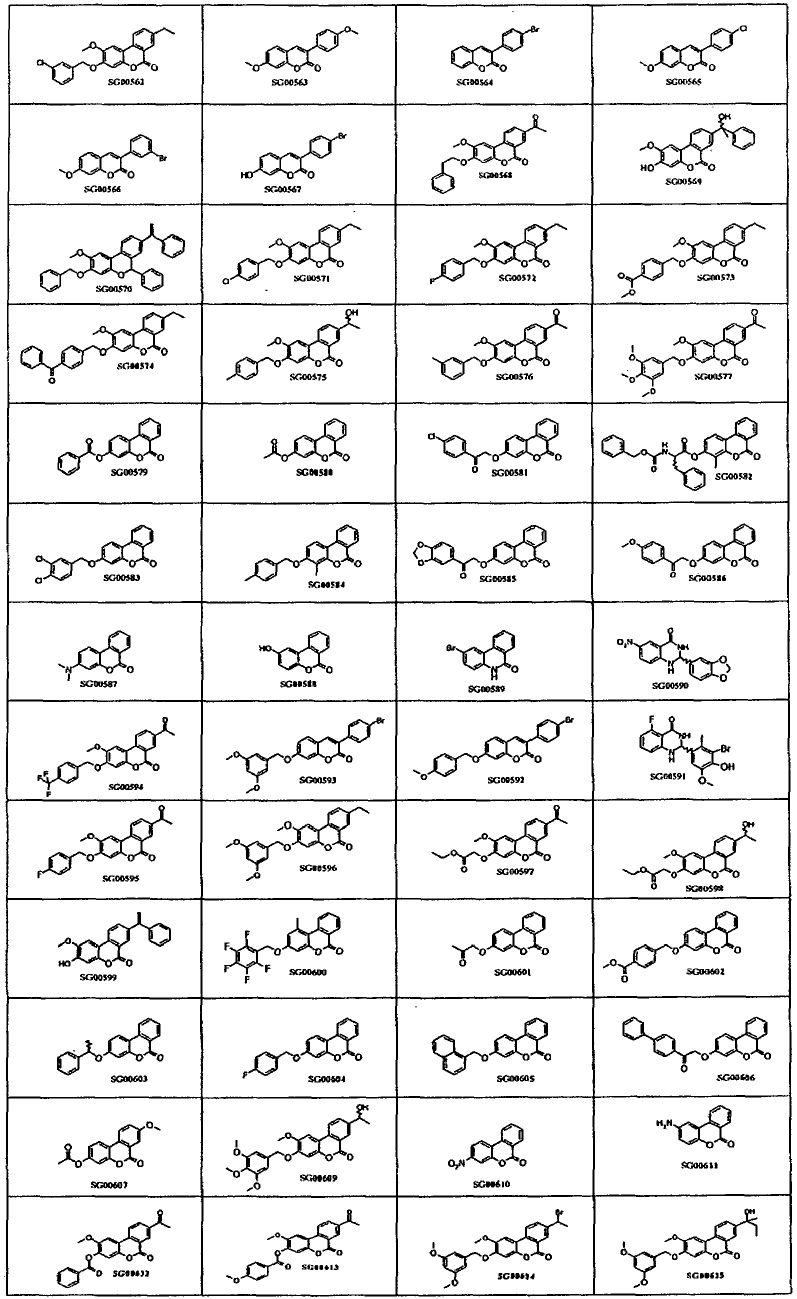

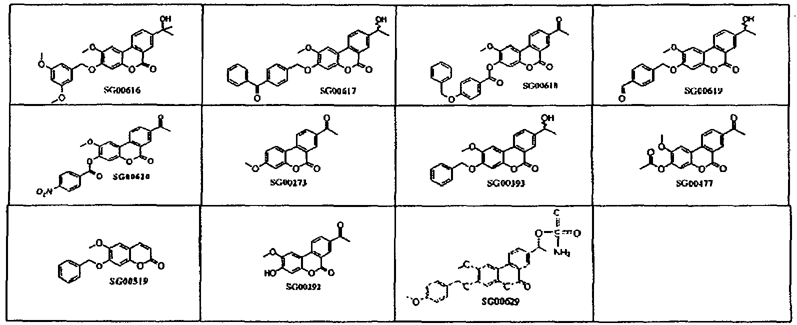

The individual benzo[c]-chromen-6-one derivatives of Table 2 are identified by the designation "SG" followed by a number. They are alternatively referred to herein by the designation "Palomid" followed by the same number, *i.e.* the terms "SG" and "Palomid" are used interchangeably throughout this application.

The compounds of Table 2 exhibit anti-fibrotic activities.

The present invention also relates to implants or other devices comprised of one or more compositions described herein or prodrugs thereof wherein the composition or prodrug is formulated in a biodegradable or non-biodegradable format for sustained release. Non-biodegradable formats release the drug in a controlled manner through physical or mechanical processes without the format being itself degraded. Bio-degradable formats are designed to gradually be hydrolyzed or solubilized by natural processes in the body, allowing gradual release of the admixed drug or prodrug. Both bio-degradable and non-biodegradable formats and the process by which drugs are incorporated into the formats for controlled release are well known to those skilled in the art. These implants or devices can be implanted in the vicinity where delivery is desired, for example, at the site of aberrant extracellular matrix.

Described herein are also conjugated prodrugs and uses thereof. More particularly, described herein are conjugates of benzo[c]chromen-6-one derivatives and the use of such conjugates in the prophylaxis or treatment of conditions associated with uncharacteristic extracellular matrix formation.

Described herein is a conjugated prodrug of a benzo[c]chromen-6-one derivative conjugated to a biological activity modifying agent, e.g., a peptide, an antibody or fragment thereof, or *in vivo* hydrolysable esters, such as methyl esters, phosphate or sulfate groups, and amides or carbamates. Modifications can include modifying a hydroxyl group with a phosphate group. This derivative would not be expected to have activity due to the modification causing a significant change to the derivative thereby losing biological activity. However the modification imparts better solubility characteristics, *i.e*., more water soluble, which could facilitate transport through the blood or give it better oral availability to allow it to reach its site of activity. Once it gets into the microenvironment where it is needed to have activity, the modification is cleaved through natural processes, *i.e.*, endogenous enzymes which are present at the site of needed activity. Alternatively it may just keep the derivative in a state to give it better systemic concentration which would then be cleaved again in the systemic circulation and thereby enhance its activity. The incorporation of benzo[c]chromen-6-one derivatives into a disease-dependently activated prodrug enables significant improvement of potency and selectivity in treating one or more disease conditions referred to hereinabove.

The pharmaceutical composition of this invention may also contain or be co-administered (simultaneously or sequentially) with one or more pharmacological agents of value in treating one or more disease conditions referred to hereinabove.

It is herein described that, the benzo[c]chromen-6-one derivatives or prodrugs thereof may be incorporated into bio-degradable or non-degradable formats allowing for sustained release. For example, the formulation being implanted in the proximity of where the delivery is desired, at the site of a tumor or in the vicinity of aberrant vasculature. Alternatively, the pharmaceutical formulation can be packaged into a delivery vehicle that has a chemical moiety that provides for specificity. For example, the moiety can be an antibody or some other such molecule that directs and facilitates delivery of the active agent to the desirable site.

Disclosed herein is also the use of the benzo[c]chromen-6-one derivatives or prodrugs thereof for the preparation of a medicant for the prophylaxis or treatment of conditions associated with any disease characterized by uncharacteristic formation of fibrotic tissue, i.e. aberrant extracellular matrix formation.

The present invention also relates to the provision of a pharmaceutical composition comprising SG 00529 according to the present invention together with a pharmaceutical acceptable carrier, diluent or excipient.

The pharmaceutical composition may also be used for the prophylaxis or treatment of conditions associated with fibrotic diseases or disorders. The present invention also pertains to methods of prophylaxis or treatment of a condition associated with a pulmonary fibrotic disease or disorder characterized by uncharacteristic extracellular matrix formation, said method including administering to a subject in need of such prophylaxis or treatment an effective amount of SG 00529 according to the present invention as described hereinabove. It should be understood that prophylaxis or treatment of said condition includes amelioration of said condition.

By "effective amount" it is meant a therapeutically effective amount that relieves symptoms, partially or completely, associated with a particular disease or syndrome. Such amounts can be readily determined by an appropriately skilled practitioner, taking into account the condition to be treated, the route of administration, and other relevant factors - well known to those skilled in the art. Such a person will be readily able to determine a suitable dose, mode and frequency of administration.

Pharmaceutically acceptable salts of SG 00529 may be prepared in any conventional manner. *In vivo* hydrolysable esters, for example, methyl esters, phosphate or sulfate groups, and amides or carbamates may be prepared in any conventional manner.

SG 00529 can be provided as physiologically acceptable formulations using known techniques and these formulations can be administered by standard routes. The compositions may be administered through means including, but not limited to, topical, oral, rectal or parenteral, for example, intravenous, subcutaneous or intramuscular, route. In addition, the compositions may be incorporated into formats allowing for sustained release, the formats being implanted in the proximity of where the delivery is desired, for example, at the site of the skin disease or aging skin or in the vicinity of aberrant vasculature. The dosage of the composition will depend on the condition being treated, the particular derivative used, and other clinical factors such as weight and condition of the subject and the route of administration of the compound - all of which is appreciated by those skilled in the art. For example, a person skilled in the art will be able by reference to standard texts, such as Remington's Pharmaceuticals Sciences 17th edition, determine how the formulations are to be made and how these may be administered.

The formulations including, but not limited to, those suitable for oral, rectal, nasal, inhalation, topical (including, but not limited to, dermal, transdermal, buccal and sublingual), vaginal or parenteral (including, but not limited to, subcutaneous, intramuscular, intravenous, intradermal, intraocular (including, but not limited to, intravitreal, sub-conjunctival, sub-Tenon's, trans-scleral), intra-tracheal and epidural) and inhalation administration. The formulations may be conveniently presented in unit dosage form and may be prepared by conventional pharmaceutical techniques. Such techniques include the step of bringing into association the active ingredient and a pharmaceutical carrier(s) or excipient(s). The formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil emulsion, *etc.*

A tablet may be made by compression or molding, optimally with one or more accessory ingredient. Compressed tablets may be prepared by compressing, in a suitable machine, the active ingredient in a free-flowing form such as a powder or granules, optionally mixed with a binder, lubricant, inert diluent, preservative, surface-active or dispersing agent. Molded tablets may be made by molding, in a suitable machine, a mixture of the powdered compound moistened with an inert liquid diluent. The tablets may optionally be coated or scored and may be formulated so as to provide a slow or controlled release of the active ingredient therein.

Formulations suitable for administration via the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutical, cosmeceutical or cosmetic acceptable carrier. A viable delivery system is a transdermal patch containing the ingredient to be administered.

Formulations for rectal administration may be presented as a suppository with a suitable base comprising, for example, cocoa butter or a salicylate.

Formulations suitable for nasal administration, wherein the carrier is a solid, include a coarse powder having a particle size, for example, in the range of 20 to 500 microns which is administered in the manner in which snuff is taken, for example, by rapid inhalation through the nasal passage from a container of the powder held close up to the nose. Suitable formulations include wherein the carrier is a liquid for administration, as for example a nasal spray or as nasal drop, including aqueous or oily solutions of the active ingredient.

Formulations suitable for vaginal administration may be presented as pessaries, tampons, creams, gels, pastes, foams or spray formulations containing, in addition to the active ingredient, ingredients such as carriers as are known in the art to be appropriate.

Formulation suitable for inhalation may be presented as mists, dusts, powders or spray formulations containing, in addition to the active ingredient, ingredients such as carriers as are known in the art to be appropriate.

Formulations suitable for parenteral administration include aqueous and non-aqueous sterile injection solutions which may contain anti-oxidants, buffers, bacteriostatic agents and solutes which render the formulation isotonic with the blood of the intended recipient; and aqueous and non-aqueous sterile suspensions which may include suspending agents and thickening agents. The formulations may be presented in unit-dose or multidose containers, for example, sealed ampules and vials, and may be stored in a freeze-dried, lyophilized, conditions requiring only the addition of the sterile liquid, for example, water for injections, immediately prior to use. Extemporaneous injection solution and suspensions may be prepared from sterile powders, granules and tablets of the kinds previously described.

Acceptable unit dosage formulations are those containing a daily dose or unit, daily sub-dose, as herein above recited, or an appropriate fraction thereof, of the administered ingredient.

In addition to the ingredients mentioned above, the formulations of the present invention may include other agents conventional in the art having regard to the type of formulation in question, for example, those suitable for oral administration may include flavoring agents.

The present invention includes compositions of about 100% to about 90% pure isomers. In another aspect, the invention pertains to compositions of about 90% to about 80% pure isomer. In yet another aspect, the invention pertains to compositions of about 80% to about 70% pure isomer. In still another aspect, the invention pertains to a composition of about 70% to about 60% pure isomer. In yet a further aspect, the invention pertains to a composition of about 60% to about 50% pure isomer. However, a steriochemical isomer labeled as alpha or beta may be a mixture of both in any ratio, where it is chemically possible by one skilled in the art. Additionally, included by this invention are both classical and non-classical bio-isosteric atom and substituent replacements and are well known by one skilled in the art. Such bio-isosteric replacements include, for example, substitution of =S or = NH for =O.

Known compounds that are used in accordance with the invention and precursors to novel compounds according to the invention can be purchased from commercial sources, for example, Sigma-Aldrich. Other compounds according to the invention can be synthesized according to known methods well known to those skilled in the art.

### Example 1 (Not part of the invention): Müller Cell Reactivity and Photoreceptor cell death is Reduced Following Experimental Retinal Detachment using an Inhibitor of the Akt/mTOR pathway

### Tissue preparation

Retinal detachments were created in adult New Zealand Red pigmented rabbits as described in Eib1 et al. (Eib1 KH, et al. The effect of alkylphosphocholines on intraretinal proliferation initiated by experimental retinal detachment. Invest Ophthalmol Vis Sci. 2007; 48:1305-11). Briefly, intramuscular injections of the combined drugs xylazine (3 mg/kg) and ketamine (15 mg/kg) were used for anesthesia and analgesia. Additional analgesia was provided by topical eye drops of proparacaine. A 0.25% solution of sodium hyaluronate (Healon; Pharmacia, Piscataway, NJ, in balanced salt solution (BSS; Alcon, Ft. Worth, TX) was infused via a glass pipette between the neural retina and RPE. The Healon is necessary to prevent spontaneous reattachment of the retina and 0.25% is the most dilute solution that maintains the detachment for extended periods. The pipette, with an external diameter of approximately 100 µm, was inserted into the eye via an incision that was made several millimeters below the pars plana to prevent the pipette from touching the lens. Approximately one-half of the inferior retina was detached in the right eye, leaving the superior attached regions as internal controls. The left eyes served as uninjected controls. Immediately after producing the detachment, 600 µg of Palomid 529 in 50 µl balance salt solution (BSS) was injected intravitreally. On day 3 after detachment, the experimental (right) eyes were given an intravitreal injection of 10 µg BrdU (Sigma, St Louis, MI) in 50 µl BSS. BrdU was given intravitreally on day 3 for both the 3-day and 7-day detachment experiments since it has been shown previously that the proliferative response is at its maximum at this time. Attached retinal regions within the eyes with the detachment served as control retinas labeled with BrdU. Animals were euthanized either 4 hr after the BrdU injection on day 3, or on day 7 using sodium pentobarbital (120 mg/ ml, IV). Three animals were used in both 3 and 7 day experiments. Following enucleation, the eye was fixed in 4% paraformaldehyde for at least 24 hr (in 0.1M sodium cacodylate buffer, pH 7.4; Electron Microscopy Sciences, Fort Washington, PA). To determine potential toxic effects of the drug, six additional animals received either vehicle (BSS, n=3) or drug injections (n=3) into normal eyes. These animals were euthanized on day 15, and the eyes were fixed in 4% paraformaldehyde and the whole eyes embedded in paraffin for light microscopic evaluation. All experimental procedures conformed to the ARVO statement for the Use of Animals in Ophthalmic and Vision Research and the guidelines of the Animal Resource Center of the University of California, Santa Barbara.

### Immunocytochemistry/ Light Microscopy

The immunocytochemistry was performed as described in Eibl et al. with slight modifications. Pieces of retinal tissue approximately 3 mm square were excised from 3 detached regions from within each eye. The tissue was rinsed in phosphate buffered saline (PBS), embedded in low-melt agarose (5%; Sigma, St. Louis, MI) and sectioned at 100 µm using a vibratome (Technical Products International, Polysciences, Warrington, PA). Sections were incubated in normal donkey serum (1:20) in PBS, 0.5% bovine serum albumin, 0.1% triton X-100, and 0.1% azide (=PBTA) overnight at 4°C on a rotator. The following day the sections were pre-treated with 2 N HCL for 1 hr as an antigen retrieval step for the BrdU. After rinsing in PBTA, the primary antibodies and lectin were added and incubated overnight at 4°C on a rotator in PBTA. Anti-BrdU (1:200, Accurate Chemical and Scientific Corp., Westbury, NY) was used to detect dividing cells, anti-vimentin (1:500; Dako, Carpinteria, CA) was used to identify Müller cells, and the isolectin B4, Griffonia Simplicifolia (1:50; Vector Labs, Burlingame, CA) was used to label microglia and macrophages. Following rinsing of the primary antibodies in PBTA, the secondary antibodies (streptavidin CY5, donkey anti-rat CY3, and donkey anti-mouse CY2; Jackson ImmunoResearch, West Grove, PA) were added together, each at 1:200 in PBTA, overnight at 4°C on a rotator. The next day the sections were rinsed in PBTA, mounted on glass slides using 5% n-propyl gallate in glycerol with the nuclear stain Hoescht (1:5000; Invitrogen, Carlsbad, CA) and viewed on an Olympus Fluoview 500 laser scanning confocal microscope. Paraffin sections of whole eyes were cut at 4 µm, counter-stained with Hemotoxylin & Eosin (H&E) and photographed using a digital camera on an Olympus BX60 microscope. Since whole eyes from 3 different animals within each group were embedded and sectioned, examination of the entire length of the retina was performed in a single section and similar retinal regions from control and experimental animals were chosen to photograph.

### Quantitation

At least 60 single plane confocal images at 1024x1024 pixel resolution were captured from 3 animals within each of the experimental groups (vehicle and drug treated). Similar retinal locations were examined in all animals. To quantify the effect of drug treatment on proliferation, the number of anti-BrdU labeled Müller cell nuclei was determined per millimeter of retina using the confocal images from both 3 and 7-day time-points. To quantify the effect of drug treatment on subretinal glial scar formation, scars were defined as areas of continuous cellular growth located sclerad to the OLM that were also labeled with anti-vimentin. The number of scars was counted and their length measured per mm of retina in the 7 day animals since no scars were observed at 3 days. To determine the width of the ONL, sections were stained with a fluorescent nuclear dye (Hoescht) and the ONL was automatically segmented by a statistic-based clustering method (Byun J, Ph.D. thesis, "Quantitative analysis and modeling of confocal retinal images". Univ. Calif. Santa Barbara, June 2007). The ONL widths in the control retina were then compared to the widths in the vehicle and drug injected eyes. Only the 7-day time-point was examined since more photoreceptor cell death has occurred at this time compared to day 3. The same sections were used to quantify all of the responses described above.

### Analysis of Attached Retinas

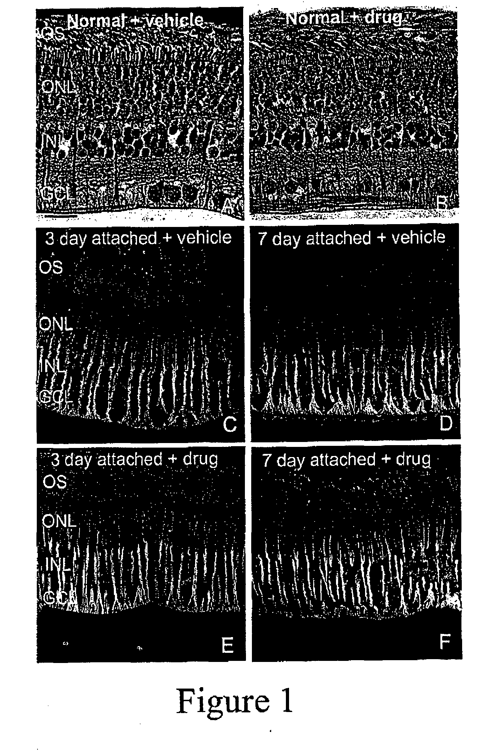

Light microscopy of paraffin embedded normal retinas and confocal microscopy of attached retinal regions labeled with various antibodies from within the detached eyes were examined to determine possible adverse affects of the drug (Fig. 1). No difference in retinal organization between vehicle and drug treated normal retinas were observed in the paraffin sections (Fig. 1A,B). In addition, the anti-vimentin and isolectin B4 labeling appeared similar between the vehicle and drug treated attached retinas at both the 3- and 7-day time-points; anti-vimentin labeling (green) extended within Müller cells from the ganglion cell layer (GCL) into the outer nuclear layer (ONL), and microglia (blue) were present only in the inner portion of the retina in all groups (Fig. 1C-F). The labeling pattern is similar to that observed in an untouched normal eye, the only difference being the presence of macrophages in the vitreous in both vehicle and drug injected eyes. Finally, no anti-BrdU labeling was observed in either the vehicle or drug treated retinas. These data indicate that the drug did not cause gross adverse affects to the morphology of the retina nor activate Muller cells nor microglia.

### Analysis of Detached Retinas

Figures 1A-1F are photographs of control attached retinas. A, B. Light micrographs of H & E stained paraffin sections illustrating the normal retinal morphology in both the vehicle (A) and drug (B) injected normal eyes. C-D. Laser scanning confocal images of attached retinal regions from within the eyes of animals with 3 or 7 day detachments and receiving an intraocular injection of either the vehicle (C,D) or drug (E,F). Sections were labeled with anti-vimentin (Muller cells, green), anti-BrdU (dividing cells, red) and isolectin B4 (microglia and macrophages, blue). Anti-vimentin labeling extended from the GCL into the ONL, no anti-BrdU labeling was observed, and the isolectin B4 labeled the fine processes of microglia in the inner retina as well as macrophages in the vitreous (the blue labeling of the OS is non-specific). OS, outer segments; ONL, outer nuclear layer; INL, inner nuclear layer; GCL, ganglion cell layer. Bar, 20 µm.

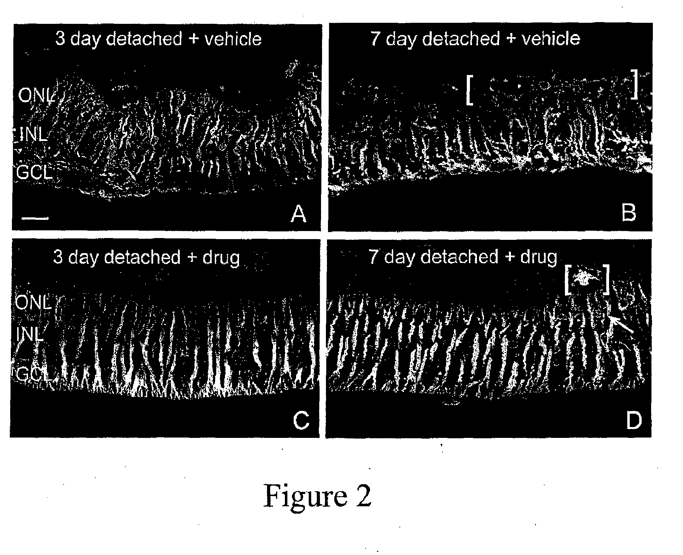

Figures 2A-2D are photographs of laser scanned confocal images of experimental detached retinas. Laser scanning confocal images of retinal sections labeled with anti-vimentin (Muller cells, green), anti-BrdU (dividing cells, red) and isolectin B4 (microglia and macrophages, blue). (A) In the 3-day detachments injected with vehicle, anti-vimentin labeling extended through the ONL to the outer limiting membrane, anti-BrdU labeling was present primarily in Muller cell nuclei in the inner nuclear layer (INL), and isolectin B4 labeling was present in microglia throughout the retina as well as in macrophages in the subretinal space. (B) In the 7-day detachments injected with the vehicle, anti-vimentin labeling of Müller cells extended into the suberetinal space (brackets), anti-BrdU labeling was present in nuclei scattered throughout the retina and in the subretinal space, and isolectin B4 labeling appeared in numerous microglia in the retina and macrophages in the subretinal space. In both the 3-day and 7-day Palomid 529 treated retinas (C,D), anti-vimentin labeling extended through the ONL and only occasionally into the subretinal space (bracket, D), anti-BrdU labeling was low, and the isolectin B4 labeling was present throughout the retina and subretinal space. Occasional microglial cells can be seen labeled with anti-BrdU and the lectin (arrow, D). Note that the drug treated detached retinas (C,D) also retained a more normal morphology by comparison to the vehicle treated detached retinas. ONL, outer nuclear layer; INL, inner nuclear layer; GCL, ganglion cell layer. Bar, 50 µm.

In detached retinal regions from the eyes injected with the vehicle at day 3, anti-vimentin labeling (green) extended to the outer limiting membrane (OLM), giving the Müller cells a thickened, distorted appearance within the retina (Fig. 2A; compare to 3 day attached retina + vehicle, Fig. 1C). No Müller cell growth was observed into the subretinal space at this time. Isolectin B4-labeled microglia (blue) appeared rounded and were dispersed throughout the retina, whereas macrophages (blue) were observed only in the subretinal space. Most anti-BrdU labeled Müller cell nuclei (red) occurred in the inner nuclear layer (INL; Fig. 2A). At 7 days, the anti-vimentin labeled Müller cell processes frequently extended into the subretinal space (bracket; Fig. 2B). The anti-BrdU labeled nuclei ("born" on day 3) generally occurred in the outer retina (i.e. distal to the INL) and in the subretinal glial scars.

At 3 and 7 days after detachment in the drug treated eyes, the occurrence of anti-BrdU labeled nuclei was greatly reduced (Fig. 2C,D). Anti-vimentin labeling was somewhat elevated when compared to non-detached controls and the Müller cells appeared thickened (compare with Fig. 1E,D) but they rarely extended into the subretinal space at either time-point; if they did, they were much smaller in size (bracket, Fig. 2D). In addition, the Müller cells did not show the distorted appearance and lateral branching characteristically observed in vehicle injected control detachments perhaps contributing to the overall more organized appearance of the retina. Certainly the retinas did not show any evidence of pathology beyond that associated with detachment. Finally, there were fewer microglia and macrophages in the drug treated eyes (Fig. 2C,D).

### Quantitation

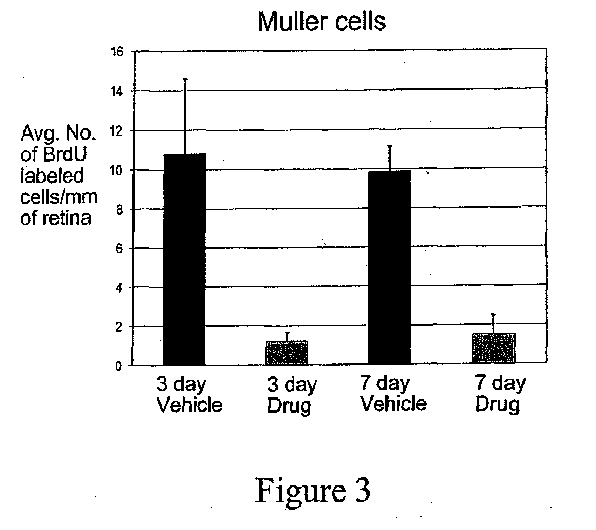

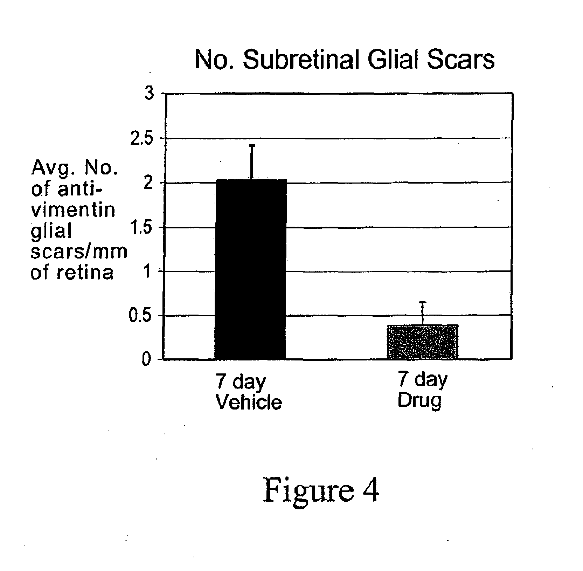

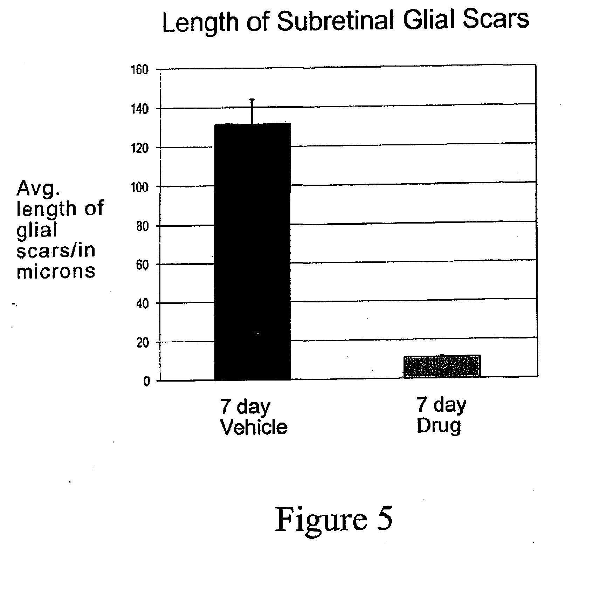

At the 3-day detachment time-point, the average number of anti-BrdU labeled Müller cell nuclei was significantly reduced from 10.79/mm of retina, in the vehicle treated control eyes, to 1.19 following drug treatment (Fig. 3). At the 7-day time-point, the number was reduced from 9.83 /mm of retina in the controls, to 1.49 following drug treatment (Fig. 3). The average number of subretinal scars was reduced from 2.03/mm of retina in controls, to 0.39 in drug treated retinas (Fig. 4). Only the 7-day time-point was quantified since there were no scars present at 3-days in either the control or drug treated animals. The average length of subretinal scars in the control eyes at day 7 was 131.28 µm and this was reduced to 11.01 µm in drug treated eyes (Fig. 5).

Specifically, Figure 3 is a bar graph illustrating the number of Müller cells proliferating after retinal detachment. The average number of anti-BrdU labeled Müller cells per millimeter of retina decreased significantly following drug treatment both at the 3 and 7-day detachment time-points compared to control detachments. Error bars, SD.

Specifically, Figure 4 is a bar graph illustrating the number of subretinal glial scars after retinal detachment. The average number of anti-vimentin labeled subretinal glial scars per millimeter of retina decreased significantly in the drug treated retinas at 7 days following detachment compared to vehicle treated retinas. Error bars, SD.

Specifically, Figure 5 is a bar graph illustrating the length of subretinal glial scars after retinal detachment. The average length of anti-vimentin labeled subretinal scars decreased significantly in the drug treated retinas at 7 days following detachment compared to vehicle treated retinas. Error bars, SD.

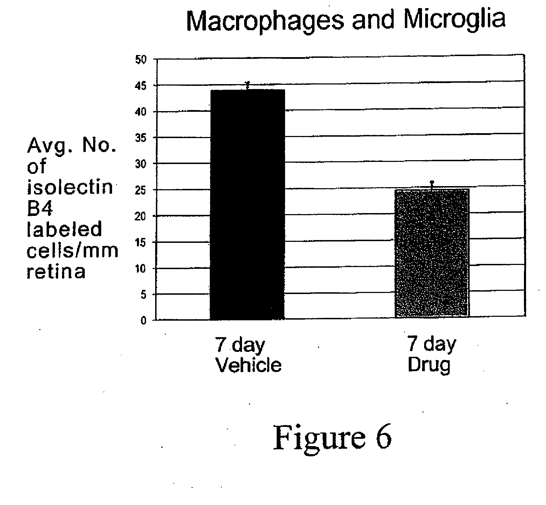

Few immune-related cells (microglia and macrophages) incorporated BrdU in either control or treated retinas, therefore, to quantify this response, the total number of isolectin B4 labeled cells were counted per millimeter of retina. At the 7-day time-point, the total number of cells decreased from an average of 43.9/mm of retina in control eyes, to 24.5/mm retina in drug treated retinas (Fig. 6).

Specifically, Figure 6 is a bar graph illustrating the number of immune-related cells after retinal detachment. The average number of isolectin B4 labeled macrophages and microglia per millimeter of retina decreased significantly in the drug treated retinas at 7 days following detachment compared to vehicle treated retinas. Error bars, SD.

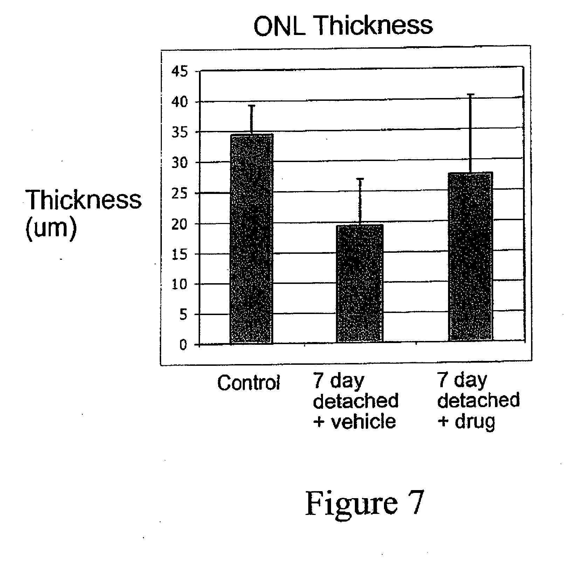

Since the outer retina appeared more organized in the drug treated eyes, as shown by the anti-vimentin labeling of Müller cells (compare Fig. 2A,B with Fig. 2C,D), we sought to determine if more photoreceptors were present by measuring the ONL width. Seven days of detachment resulted in a statistically significant decrease in the average thickness of the ONL from 34.45 µm in the normal retina to 19.40 µm in the vehicle treated detached retinas (p<1.27 E-11; Fig. 7). There was also a statistically significant decrease in the thickness of the ONL in the drug treated detached retinas to 27.21 µm when compared to normal attached retina (p<0.001). However, the thickness of the ONL was significantly greater in the detached retinas treated with drug than those treated with vehicle (27.21 µm vs. 19.40 µm; p<8.81 E-06) showing that Palomid 529 exerts some rescue effect on photoreceptors after detachment.

Specifically, Figure 7 is a bar graph illustrating the average thickness of the outer nuclear layer (ONL) after detachment. The ONL in 7 day detached retina treated with Palomid 529 was thicker than in 7 day detached retina treated with vehicle, but the ONL in both detachment groups was significantly reduced by comparison to control retinas. Error bars, SD.

Two potentially blinding conditions that can result from retinal detachment are PVR and subretinal fibrosis, the formation of cellular membranes on the vitreal or photoreceptor surface of the retina. Currently the only treatment is surgical removal of the membranes. This, however, involves surgical intervention and is often not a permanent solution since recurrence of secondary membranes is not uncommon. Ideally a pharmacological adjunct could be given at the time of reattachment surgery to prevent membrane formation or alternatively at the time of membrane removal to prevent continued growth of the tissue. To date no pharmacological approaches have proven clinically effective at inhibiting this process. In the current invention a single injection of Palomid 529 is effective in an animal model at reducing the proliferation induced by detachment, the subsequent hypertrophy and growth of the Müller cells into the subretinal space, and the activation of microglia. In addition, there is no apparent toxicity associated with intraocular delivery of the drug and, in fact, the ONL thickness was greater in the Palomid 529 treated detached retinas, inidcating some neuroprotective effect on photoreceptors. Hence this drug is useful for treating a variety of related human retinal diseases in which proliferation and/or photoreceptor cell death are components.

Typically, PVR is thought to be a condition involving undesirable cell proliferation, cell spreading and contractility. Indeed, dividing cells have been observed in membranes removed from patients with PVR. However, data from animal models suggest that in addition to proliferation, the growth and hypertrophy of Müller cells plays a role in the response perhaps by providing a cellular scaffold on which more complex membranes, involving a variety of cell types, can grow. Both proliferation and hypertrophy of Müller cells begin within the first few days after detachment. After 1 week, proliferation declines to low levels but processes from these cells continue expanding in the subretinal space, apparently as long as the retina remains detached. This is also true for the growth of epiretinal membranes in the vitreous. Müller cell growth on the vitreal surface appears to begin, however, with retinal reattachment, not detachment. Reattachment reduces the proliferative response significantly, but the growth of membranes along the vitreal retinal surface continues since large membranes are generally observed weeks to months after reattachment. The fact that PVR and subretinal fibrosis are complex combinations of proliferation and cell growth may explain why drugs that target solely proliferation such as 5-fluorouracil, were not effective at reducing epiretinal membrane formation in human patients. The Akt/mTOR pathway, through which Palomid acts, has been shown to regulate both proliferation and cell spreading. This may provide a mechanism for Palomid's dramatic reduction of both proliferation and subretinal gliosis in the rabbit model of detachment although its precise mechanism of action is not known. It is presumed that Palomid is acting directly on Müller cells since these are the most numerous cell types undergoing division. Palomid 529's effect on increased photoreceptor survival could be direct or indirect. It has been shown that activation of the PI3K/Akt signaling pathway can have a neuroprotective effect in the retina by inhibiting light-induced photoreceptor apoptosis indicating that the drug may in fact have a direct effect on photoreceptors.

Palomid 529 has an expected half-life of at least 3 months in the vitreous, enabling the observation of an effect on proliferation and scar formation 7 days after detachment. The rapid clearance of drugs such as 5-fluorouracil may contribute to its ineffectiveness in preventing PVR. While the average number of subretinal scars was reduced approximately 4 fold with the addition of Palomid 529, their length was reduced more than 20 fold. This indicates that the drug not only reduced Müller cell growth into the subretinal space, but also the actual expansion of the processes that do reach that space. This correlates with the observation that Muller cells within the retina appear less branched and tortuous in the treated eyes (see Fig. 2C,D), indicating a result of Palomid's effect on cell spreading, in this case translated into cell hypertrophy. Clinically, Palomid 529 can prevent the formation of scars and also reduce the overall growth of scars that are already present. Finally, Palomid 529 also reduced the microglial/macrophage response, and since these cells become highly migratory when activated, this may also be a result of the anti-spreading effect of the drug.

In summary, the data presented herein indicate that Palomid 529 is an effective agent for decreasing proliferation and glial cell growth, as well as photoreceptor cell death induced by retinal detachment, and therefore represents a novel way to improve the overall outcome of repairing rhegmatogenous retinal detachments.

### Example 2 (Not part of the invention): Müller Cell Proliferation and Glial Scar Formation is Reduced Following Experimental Retinal Detachment Using Palomid 529 an Inhibitor of the Akt/mTOR Pathway.

**Methods:** Experimental retinal detachments were made in the right eyes of pigmented rabbits. Six hundred micrograms of Palomid 529 in 50 microliters of PBS, or PBS alone was injected intravitreally on day 0, immediately after retinal detachment. Each rabbit received 10 micrograms of BrdU intravitreally on day 3. Animals were sacrificed on day 3 or 7, at which time the tissue was fixed in paraformaldahyde, embedded in agarose and sectioned at 100 microns. The sections were labeled with anti-BrdU, to detect dividing cells, and anti-vimentin, to identify Muller cells. Labeling was imaged on an Olympus Fluoview confocal microscope, and the resultant digital images were analyzed to determine the number of proliferating cells as well as the number and length of subretinal glial scars.

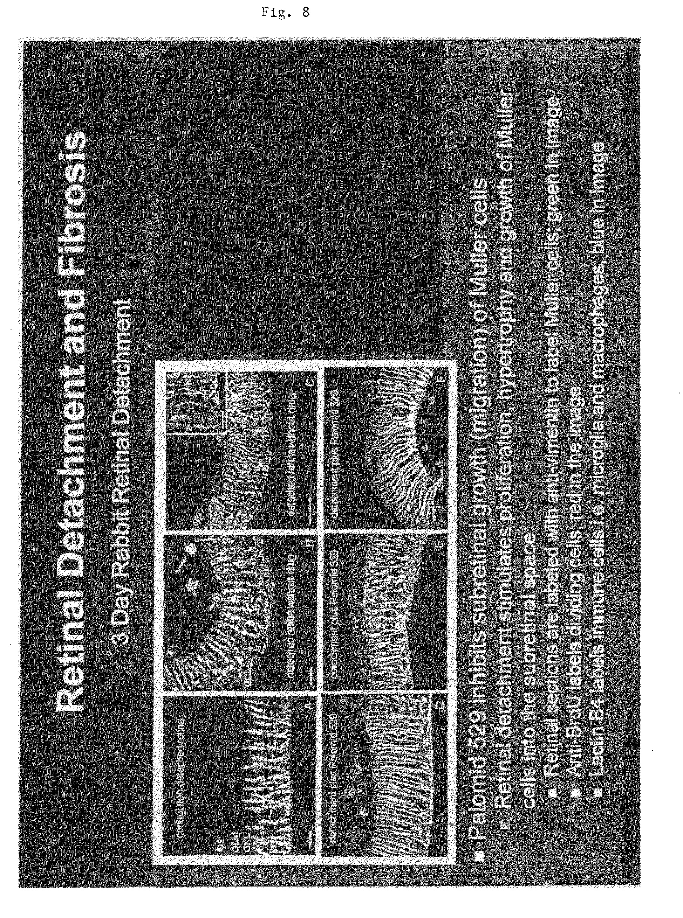

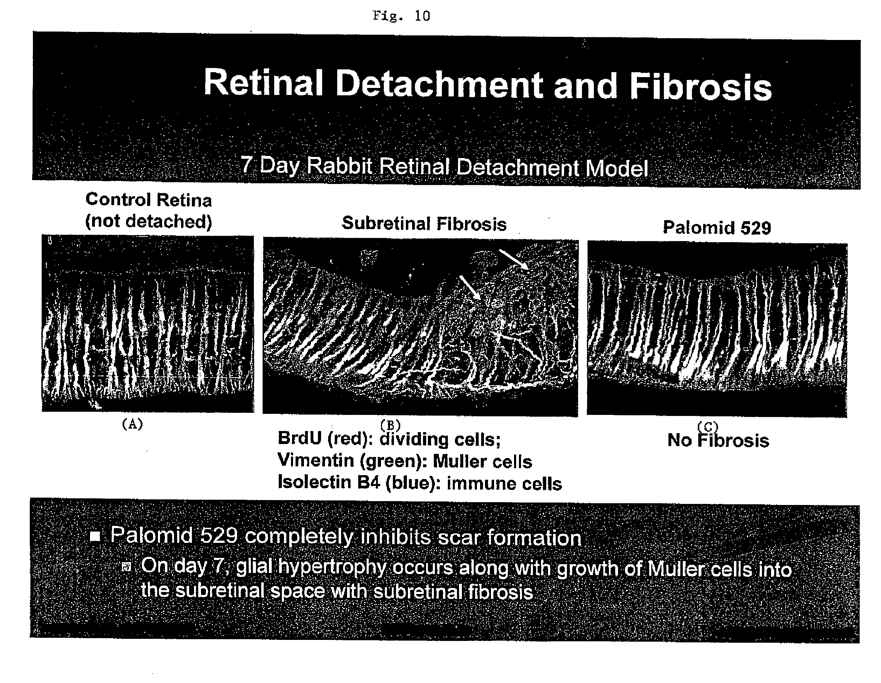

**Results:** In control detachments, Muller cells make up the majority of BrdU labeled cells, and are the cells that form glial scars by growing into the subretinal space. The data show a statistically significant decrease in the number of BrdU labeled Müller cells per millimeter at both the 3 (Figures 8A-8F) and 7(Figures 10A-10C) day time points. No glial scars were observed at the 3 day time point in either control or drug treated animals, however, there was a significant decrease in the number and size of sub-retinal scars at day 7.

Conclusions: The data indicate that Palomid 5299 is an effective suppressor of Muller cell proliferation and glial scar growth in a rabbit model of retinal detachment. Therefore, inhibiting the Adt/mTOR signal transduction pathway is a strategy to decrease proliferation induced by detachment and represents a novel therapy for related human diseases such as proliferative vitreoretinopathy.

### Example 3: Effect of Palomid 529 on Mediators of Differentiation of and Signal Transduction in Human Lung Fibroblasts

Transforming growth factor-beta one (TGF-β1) and alpha-Thrombin (α-Thrombin) are known to be involved in the pathogenesis of fibrotic disease. In an in vitro model of pulmonary fibrosis, TGF-β1 stimulates myofibroblast differentiation of human lung fibroblasts characterized by expression of contractile smooth muscle (SM)-specific proteins such as SM-alpha-actin (SM-α-actin). TGF-β1 also stimulates SM-α-actin expression in human lung fibroblasts which parallels a profound induction of serum response factor (SRF) expression and activity. Increased contractile gene expression with SM-α-actin as marker, increased SRF, increased connective tissue growth factor (CTGF) and increased matrix genes (e.g. fibronectin) are examples of effects observed during myofibroblast differentiation. Myofibroblast differentiation is induced by stimulation with TGF-β1 thereby increasing markers of differentiation.

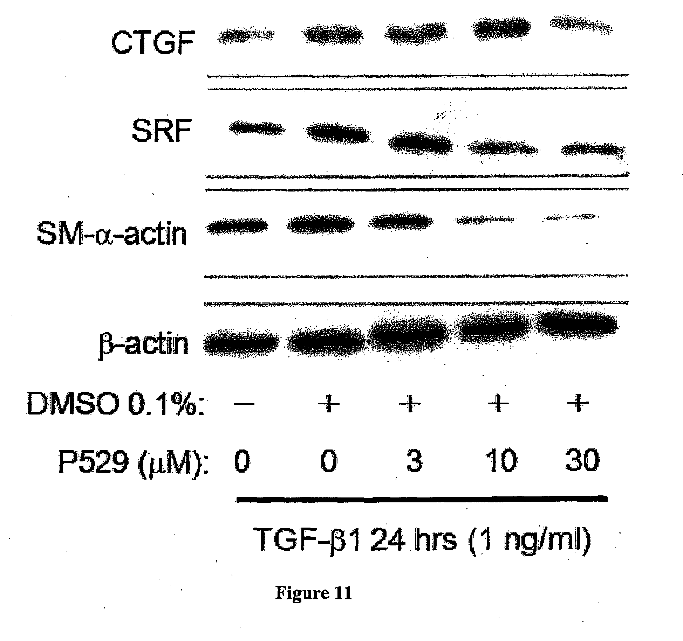

Palomid 529 inhibits TGF-β1 stimulated increases of CTGF (18% at 30 µM Palomid 529), SRF (29% at 30 µM Palomid 529) and SM-α-actin (65% at 30 µM Palomid 529) in human lung fibroblasts, see Figure 11.

Figure 11 shows the Effect of Palomid 529 on Mediators of Differentiation of Human Lung Fibroblasts. Human lung fibroblasts were pre-treated with Palomid 529 for 30 minutes prior to TGF-β1 stimulation. Western Blot with antibodies against CTGF, SRG, SM-α-actin and β-actin is shown. Scanning software, Scion Image (Scion Corporation, Frederick, MD), was used to digitize bands on Western Blot for numeric calculation of percent inhibition described in text. β-actin levels showed no significant changes in any of the samples as they all were within 11% of each other.

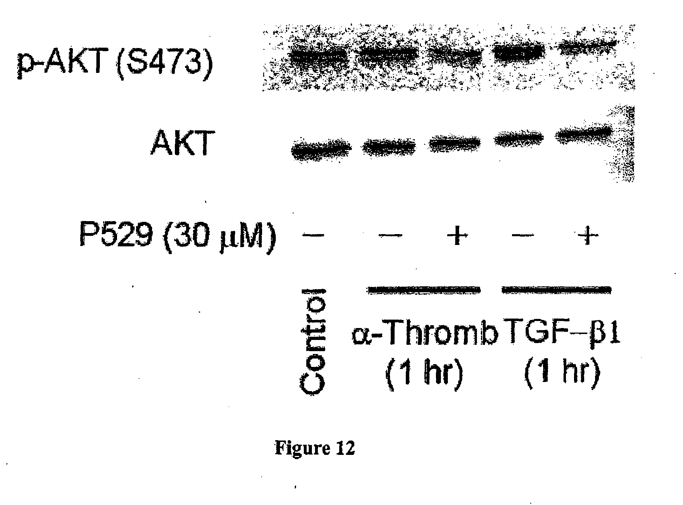

Myofibroblast activation, as shown in Figure 11, is at least in part induced through the activation of signal transduction proteins in the Akt/mTOR pathway. Although stimulation of human lung fibroblasts by α-Thrombin did not increase levels of phosphorylated AKT over that of basal levels, Palomid 529 was able to decrease both AKT phosphorylation in the presence or absence (basal level of AKT phosphorylation) of α-Thrombin by 15%. TGF-β1 is shown to stimulate AKT phosphorylation over basal levels by 19%. Palomid 529 inhibited phosphorylation of AKT stimulated by TGF-β1 by 27% including basal level of AKT phosphorylation by 13%, see Figure 12.

Figure 12 shows the Effect of Palomid 529 on Signal Transduction in Human Lung Fibroblasts. Human lung fibroblasts are stimulated for one hour with either TGF-β1 or α-Thrombin in presence of 30 µM Palomid 529 to effect activation of signal transduction proteins. p-AKT(S473) and AKT levels are measured by Western Blot. Scanning software, Scion Image (Scion Corporation, Frederick, MD), was used to digitize bands on Western Blot for numeric calculation of percent inhibition described in text. AKT levels showed no significant changes in any of the samples as they all were within 10% of each other.

## Claims

1. Use of a compound of formula 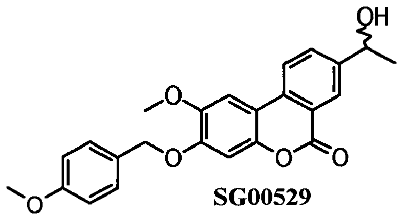 or a pharmaceutically acceptable salt thereof in the manufacture of a medicament for the therapeutic and/or prophylactic treatment of a disease selected from pulmonary fibrosis and idiopathic pulmonary fibrosis.

2. The use of claim 1, wherein said compound is

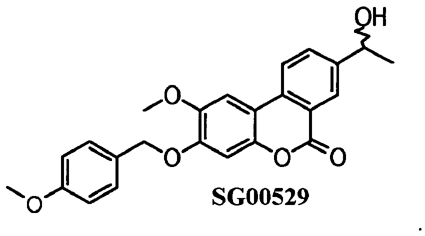

3. The use of any preceding claim, wherein said medicament further comprises an acceptable delivery vehicle.

4. The use of any preceding claim, wherein said medicament is a capsule, cachet, tablet, powder, granule, solution, suspension in an aqueous liquid or a non-aqueous liquid, oil-in-water liquid emulsion, or water-in-oil emulsion.

5. The use of any preceding claim, wherein said medicament is formulated for oral, rectal, nasal, inhalation, topical, vaginal, or parenteral administration.

6. The use of any preceding claim, wherein said medicament is associated with an implant.

7. The use of any preceding claim, wherein said medicament is associated with a device.

## Patentansprüche

1. Verwendung einer Verbindung der Formel 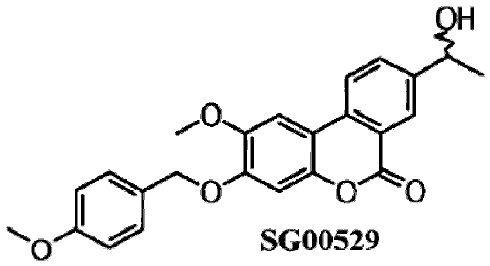 oder eines pharmazeutisch verträglichen Salzes davon bei der Herstellung eines Medikaments für die therapeutische und/oder prophylaktische Behandlung einer Erkrankung, die aus Lungenfibrose und idiopathischer Lungenfibrose ausgewählt ist.

2. Verwendung nach Anspruch 1, worin die genannte Verbindung Folgendes ist:

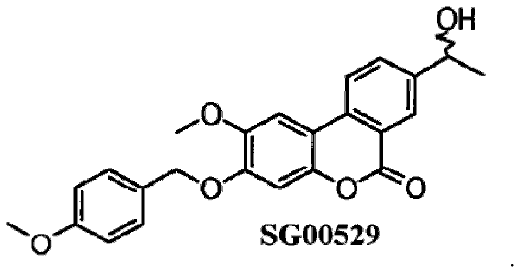

3. Verwendung nach einem vorstehenden Anspruch, worin das genannte Medikament weiter ein verträgliches Abgabevehikel umfasst.

4. Verwendung nach einem vorstehenden Anspruch, worin das genannte Medikament Folgendes ist: eine Kapsel, Kapsel aus Stärkemasse, Tablette, ein Pulver, Granulat, eine Lösung, Suspension in einer wässrigen Flüssigkeit oder einer nichtwässrigen Flüssigkeit, flüssige Öl-in-Wasser-Emulsion oder Wasser-in-Öl-Emulsion.

5. Verwendung nach einem vorstehenden Anspruch, worin das genannte Medikament für die orale, rektale, nasale, inhalative, topische, vaginale oder parenterale Verabreichung formuliert ist.

6. Verwendung nach einem vorstehenden Anspruch, worin das genannte Medikament mit einem Implantat verbunden ist.

7. Verwendung nach einem vorstehenden Anspruch, worin das genannte Medikament mit einer Vorrichtung verbunden ist.

## Revendications

1. Utilisation d'un composé de formule 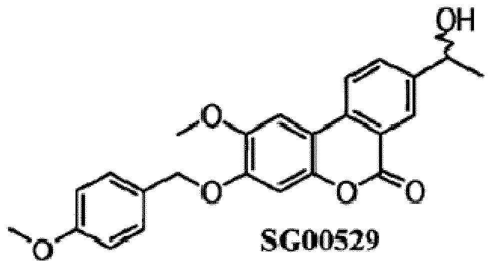 ou d'un sel pharmaceutiquement acceptable de celui-ci, dans l'élaboration d'un médicament destiné au traitement thérapeutique et/ou prophylactique d'une maladie choisie parmi la fibrose pulmonaire et la fibrose pulmonaire idiopathique.

2. Utilisation selon la revendication 1, dans laquelle ledit composé est

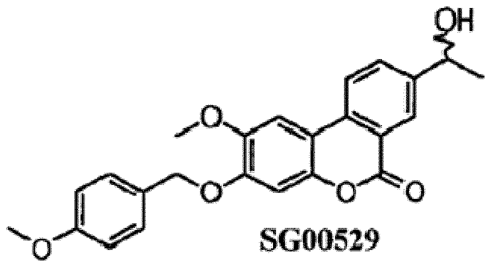

3. Utilisation selon l'une quelconque des revendications précédentes, dans laquelle ledit médicament comprend en outre un véhicule d'administration acceptable.

4. Utilisation selon l'une quelconque des revendications précédentes, dans laquelle ledit médicament est une capsule, un cachet, un comprimé, une poudre, des granulés, une solution, une suspension dans un liquide aqueux ou un liquide non aqueux, une émulsion liquide huile-dans-eau ou une émulsion eau-dans-huile.

5. Utilisation selon l'une quelconque des revendications précédentes, dans laquelle ledit médicament est formulé pour une administration orale, rectale, nasale, par inhalation, topique, vaginale ou parentérale.

6. Utilisation selon l'une quelconque des revendications précédentes, dans laquelle ledit médicament est associé à un implant.

7. Utilisation selon l'une quelconque des revendications précédentes, dans laquelle ledit médicament est associé à un dispositif.
